(19)

Europäisches
Patentamt
European
Patent Office
Office européen
des brevets

(11) **EP 2 910 245 A1**

(12) **EUROPEAN PATENT APPLICATION**

(43) Date of publication:
**26.08.2015 Bulletin 2015/35**

(51) Int Cl.:
*A61K 31/4418* (2006.01)      *A61K 47/10* (2006.01)
*A61K 47/36* (2006.01)        *A61K 9/08* (2006.01)
*A61P 31/10* (2006.01)

(21) Application number: **14156113.4**

(22) Date of filing: **21.02.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Polichem SA
1526 Luxembourg (LU)**

(72) Inventors:
• **Mailland, Federico
CH-6900 LUGANO (CH)**

• **Ceriani, Daniela
IT-21050 Besano (IT)**
• **Iob, Giuliana
CH-6953 Lugaggia (CH)**
• **Sarno, Simone
IT-21013 Gallarate (IT)**

(74) Representative: **Pistolesi, Roberto et al
Dragotti & Associati Srl
Via Nino Bixio, 7
20129 Milano (IT)**

(54) **Topical antifungal composition for treating onychomycosis**

(57)     The present invention is directed to a nail lacquer consisting essentially of ciclopirox as an antimycotic agent, hydroxypropyl chitosan as film forming agent, water and a lower alkanol as solvent. The invention is also directed to such a nail lacquer for use in treating onychomycosis.

EP 2 910 245 A1

**Description**

The present invention is directed to a nail lacquer consisting essentially of ciclopirox as an antimycotic agent, hydroxypropyl chitosan as film forming agent, water and a lower alkanol as solvent. The invention is also directed to such a nail lacquer for use in treating onychomycosis.

BACKGROUND OF THE INVENTION

Onychomycosis is an infection of the nails which represents the most common nail disease worldwide. At the beginning of the past century this fungal infection was still considered as very rare, but its prevalence increased dramatically during the last decades of the century, reaching very high rates in the US (up to 14% of the general population) and in the EU (near 30% of selected populations) (Baran R, Hay R, Haneke E, Tosti A (Eds), Epidemiology. In: Onychomycosis - the current approach to diagnosis and therapy. London, Martin Dunitz, 1999: pp. 6-9). Presently, onychomycosis represents approximately 50% of all nail disorders. It is a fungal disease of the nail mostly caused by dermatophytes, such as *Trichophyton rubrum, Trichophyton mentagrophytes and Epidermophyton floccosum,* and is far more common on the toenails than on the fingernails.

Both genders appear to be equally affected. Onychomycosis may occur at any age but it is rare prior to puberty, and an increased incidence has been reported in the elderly population. Risk factors for onychomycosis are diabetes, nail psoriasis, hyperhidrosis, impaired peripheral circulation, nail trauma, tinea pedis and immunodeficiency (Tosti A, Hay R, Arenas-Guzmán R, Patients at risk of onychomycosis - risk factor identification and active prevention. J Eur Acad Dermatol Veneorol, 2005, 19:13-16).

The pharmacological treatment of this difficult to eradicate and often recurring disease is done by oral antifungal agents or by topically administered medicated nail lacquers with antimycotic agents as active ingredients. Among antimycotic agents approved for oral administration, terbinafine is considered as the golden standard for onychomycosis worldwide, and is reported to achieve a complete cure in 38% of patients. Terbinafine is an antifungal agent provided with a strong activity on dermatophytes and molds.

Itraconazole and fluconazole are reportedly less effective. None of those drugs, terbinafine, itraconazole or fluconazole, is devoid of rare but serious, sometimes fatal adverse events (Ajit C, Suvannasankha A, Zaeri N, Munoz SJ, Terbinafine-associated hepatotoxicity. Am J Med Sci. 2003; 325:292-5; SløRdal L, Spigset O. Heart failure induced by non-cardiac drugs. Drug Saf. 2006; 29:567-86).

It is unacceptable that a patient risks life-threatening adverse reactions from a treatment of nail infections. A large medical need is still present in the management of onychomycosis, in order to find treatments able to improve the rate of effectiveness and at the same time to decrease the risk of toxicity. Topical products in form of medicated nail lacquers may solve the problem, provided they are able to put the antimycotic agent in contact with the nail for a sufficient time to let it penetrate through the nail lamina in order to be available under the nail structure, into the nail bed, where there are the hyphae of pathogenic fungi.

The attempt to formulate terbinafine in topical compositions to be applied directly on the affected areas failed to achieve the desired effect (Elewski B, Ghannoum MA, Mayser P et al. Efficacy, safety and tolerability of topical terbinafine nail solution in patients with mild-to-moderate toenail onychomycosis: results from three randomized studies using double-blind vehicle-controlled and open-label active-controlled designs. J Eur Acad Dermatol Veneorol, 2011, DOI: 10.1111/j.1468-3083.2011.04373.x).

A topical treatment with a nail lacquer based on amorolphine as antimycotic active ingredient is approved in Europe but not in the USA, but in the sole controlled randomized study presently available in the scientific literature, its efficacy in terms of complete cure rate was lower than 1% of treated patients among a population of over 500 patients with onychomycosis included in the study (Elewski B. et al. already cited). A solution of Tioconazole is approved in few countries for topical treatment of onychomycosis, but no controlled randomized study does exist for this product, thus its efficacy is not known.

The only commercially available antimycotic agent with efficacy in the treatment of onychomycosis proved by controlled randomized studies, is ciclopirox, a hydroxy-pyridone derivative endowed with a broad spectrum of antimycotic activity (Subissi A. et al. Ciclopirox - Recent Nonclinical and Clinical Data Relevant to its Use as a Topical Antimycotic Agent. Drugs 2010; 70 (16): 2133-2152).

This antimycotic agent, or its olamine salt, has been used in different compositions disclosed for use in onychomycosis. WO00/15202 describes topical products for application onto nails which may be used for the treatment of onychomycosis. Those products are free of water and contain one or more active agents (among them ciclopirox olamine), a C1 to C4-alkyl ester of lactic acid, tartaric acid or citric acid as a carrier, at least one humectant and optionally physiologically acceptable excipients. WO/9939680 discloses an antifungal nail lacquer suitable for treatment of onychomycosis, comprising a polyacrylic acid polymer, effective amounts of ciclopirox and pharmaceutical salts thereof. That lacquer is characterized by a water insoluble film-forming polymer which protects the treated nails by formation of a hard, clear and water resistant film. In EP-A-226984 an antimycotic nail varnish is described containing a ciclopirox salt, in combination with a water insoluble film forming agent, physiologically acceptable solvents and additives. Penlac nail lacquer (PDR 2003) is a topical solution which contains 8% ciclopirox in a solution base consisting of ethyl acetate, isopropyl alcohol, and as film forming agent a butyl monoester of poly[methylvinyl ether/maleic acid] in isopropyl alcohol. Penlac nail lacquer was found superior to placebo in the treatment of onychomycosis. Gupta et al. reported two pivotal US studies performed with Penlac, having the same experimental design. The combined results of these two studies showed a 34% mycological cure versus 10% with placebo ($p < 0.001$), and a treatment cure of 7% versus 1%, respectively ($p < 0.001$). (Gupta AK, Malkin KF. Ciclopirox nail lacquer and podiatric practice. J Am Podiatr Med Assoc. 2000; 90(10): 502-7)

WO02/0768A1 discloses antimycotic nail varnish compositions containing an antimycotic agent, a water soluble polymeric film-forming agent selected from hydroxalkyl and carboxyalkyl chitosans, ethyl acetate (as penetration enhancer), cetostearyl alcohol (as plasticizer), ethanol and water. This composition achieves a better permeation of nails compared to Penlac in a human nail penetration model (Monti D, Saccomani L, Chetoni P et al. Drug Dev Ind Pharm. 2005 Jan;31(1):11-7). Efficacy in onychomycosis is about 13% of complete cure and almost 30% of responders after a 48 weeks of daily treatment followed by a 12-week follow up without treatment, resulting significantly superior to Penlac (Baran R, Tosti A, Hartmane I et al. An innovative water soluble biopolymer improves efficacy of ciclopirox nail lacquer in the management of onychomycosis. J Eur Acad Dermatol Veneorol, 2009, 23:773-781). The disadvantage is that this composition requires a penetration enhancer and a plasticizer, which flocculates at temperatures lower than 15 °C and requires special temperatures for transportation and storage.

It has now been surprisingly found that a simpler composition of ciclopirox, containing ciclopirox as the sole active antimycotic ingredient, together with a film forming agent and a proper solvent system, is effectively forming a film on the nail plate, by allowing efficient permeation of the active ingredient and potentially is equally efficacious in the treatment of onychomycosis. Furthermore, the composition appears at least as effective or even more effective when it is applied at a lower than once a day dose regimen.

## DESCRIPTION OF THE INVENTION

An object of the present invention is a composition comprising at least about 7% by weight ciclopirox or a pharmaceutically acceptable salt thereof, hydroxypropyl chitosan, a lower alkanol and water, and its use to treat onychomycosis in a patient in need of such a treatment.

A further object of the present invention is a composition suitable to treat onychomycosis in a patient in need of such a treatment, such composition consisting essentially of:

a)ciclopirox and/or at least a pharmaceutically acceptable salt thereof in an amount of from 7 to 9% by weight of the composition,
b)hydroxypropyl chitosan in an amount of from 0.1 to 4% by weight of the composition,
c)water in an amount of from 10.0 to 30.0% by weight of the composition,
d)at least a lower alkanol in an amount of from 65 to 85% by weight of the composition.

Moreover, the present invention is directed to a nail lacquer for use in treating onychomycosis by administering said composition to the affected nail(s) by a dose regimen which may be equal to or lower than once-a-day for the length of treatment, which is generally up to one year. The composition according to the present invention is simpler than that disclosed in the examples of WO02/07683A1, as it contains a lower number of ingredients. This leads to an easier, more economic, less time consuming and environment friendly manufacturing process and to a lower cost of goods that translates in a reduced cost of therapy and a reduced exposure of both the patients and the environment to the chemical agent.

Furthermore, the composition according to the present invention does not require the presence of a penetration enhancer in order for the active ingredient to efficiently penetrate into and through the nail plate, as the active ingredient, ciclopirox, was found to reach very high concentrations in the nail lamina in both in vitro and in vivo studies.

The composition according to the present invention does not contain plasticizer agents and does not flocculate at temperatures lower than 15°C, thus it does not require storage or transportation at controlled temperature. Furthermore, the composition according to the present invention does not contain cetostearyl alcohol, an excipient included in the composition used by Baran et al (2009) that may cause local skin reactions and it is the object of a warning reported on the leaflet, as stated by the European Commission in the Notice to Applicants, volume 3B (document n. ENTR/F2/BL D(2003)).

The compositions according to the present invention, simpler than those disclosed in WO02/07683A1, show a comparable stability from the chemical and technological point of view, in terms of impurities, color, viscosity and rheology. The evaporation time is comparable or improved in respect to the art, both macroscopic and microscopic appearances are better and the compositions according to the present invention do not smell bad.

The amount of component a) in the composition is in the range from 7 to 9% w/w, preferably 7.5 to 8.5% w/w, and more preferably of about 8% w/w of the total composition. The composition of the present invention also comprises hydroxypropyl chitosan, namely a water soluble film forming agent, as component b). Film forming agents are by definition (see e.g. DIN 55945 (12/1988)) components of a binder which are essential for forming a film, i.e. a thin layer or cover. The term "water soluble" means in this context that the film forming agent is fully compatible with water so that at 20°C one part of the film forming agent is soluble in 100 parts or less, preferably 50 parts or less, more preferably 30 parts or less, most preferably 10 parts or less of water.

The amount of the component b) is in the range from 0.1 to 4.0 % w/w, preferably 0.2 to 2.0% w/w, and more preferably of about 0.5 to 1.5% w/w, of the total composition.

The composition in accordance with the present invention further comprises water as component c). The amount of component c) in accordance with the present invention is from 10 to 30% w/w, more preferably from 12 to 20% w/w, of the total composition.

The composition in accordance with the present invention further comprises a lower alkanol or a mixture of lower alkanols as a solvent as component d). The lower alkanol is preferably a $C_1$-$C_4$- alkanol and may be selected from ethanol, propanol, isopropanol, or butanol.

Preferably, the total amount of lower alkanol used in combination with water present in the composition in accordance with the present invention is such to provide acceptable drying times of the formulation once applied to the nails. An acceptable drying time, i.e. the time taken to be dry by touch, is preferably less than about two minutes.

Component d) is usually employed in an amount suitable in order to impart the above noted properties. It is preferred that the component d) be present in the composition in accordance with the present invention in an amount from 65 to 85% w/w, more preferably from 70 to 80% w/w, of the total composition.

According to an embodiment of the invention, the composition consists of a) 7.5 to 8.5% by weight ciclopirox, b) 0.1 to 4.0% by weight hydroxypropyl chitosan, c) 10 to 30% by weight purified water and d) 65 to 85% by weight ethanol.

According to a further embodiment of the invention, the composition consists of a) about 8% by weight ciclopirox, b) 0.2 to 2.0% by weight hydroxypropyl chitosan, c) 12 to 20.0% by weight purified water and d) about 70 to 80% by weight ethanol.

For the purposes of the present invention, the expression "consisting essentially of" means that the claimed composition, in addition to components a), b), c) and d), may optionally contain other excipients and/or adjuvants which, however, should not be present in amounts higher than 8% w/w with respect to the composition; plasticizers and/or penetration enhancers being excluded from such additional optional excipients and/or adjuvants.

According to a further embodiment, the composition of the present invention consists of components a), b), c) and d), whose percentages therefore sum up to 100.

The composition of the present invention is illustrated, but not limited to, the following examples. All amounts in % are w/w %.

EXAMPLE 1

Batches P-14-004, P-14-014, P-14-015, P-14-016 and P-14-017 were prepared following the teaching of the present invention with the following w/w % compositions:

| Ingredient | Batch number | | | | |
|---|---|---|---|---|---|
| | P-14-004 | P-14-014 | P-14-015 | P-14-016 | P-14-017 |
| Ethanol | 73.00 | 78.00 | 78.50 | 73.00 | 78.70 |
| Ciclopirox | 8.00 | 8.00 | 8.00 | 8.00 | 8.00 |
| Purified Water | 18.00 | 13.00 | 13.00 | 18.50 | 13.00 |
| Hydroxypropyl Chitosan | 1.00 | 1.00 | 0.50 | 0.50 | 0.30 |

The formulations were prepared by mixing the ingredients using a suitable closed vessel provided with a stirrer. The resulting mixture is stirred until dissolution.

EXAMPLE 2 (comparative)

Batches P-14-003, P-14-002, P-14-001 were prepared following the disclosure of WO02/07683A1 and have the following w/w % compositions:

| Ingredient | Batch number | | |
|---|---|---|---|
| | P-14-003 | P-14-002 | P-14-001 |
| Ethanol | 73.00 | 73.00 | 73.00 |
| Cetostearyl Alcohol | 1.00 | - | 1.00 |
| Ethyl Acetate | - | 4.00 | 4.00 |
| Ciclopirox | 8.00 | 8.00 | 8.00 |
| Purified Water | 17.00 | 14.00 | 13.00 |
| Hydroxypropyl Chitosan | 1.00 | 1.00 | 1.00 |

*Preparation*

The formulations are prepared by using a suitable closed vessel provided with a stirrer. To this vessel are added ethanol, ethyl acetate, cetostearyl alcohol, ciclopirox and water to form a homogeneous mixture. Thereafter, hydroxypropyl chitosan is added and the resulting mixture is stirred until dissolution.

EXAMPLE 3

The formulations prepared according to Example 1 (batch P-14-004, P14-014, P-14-015, P-14-016 and P-14-017) and those prepared according to Example 2 (batch P-14-001 and batch P-14-003) were stored at prescribed temperatures (5°C and 25°C) for at least 1 hour.

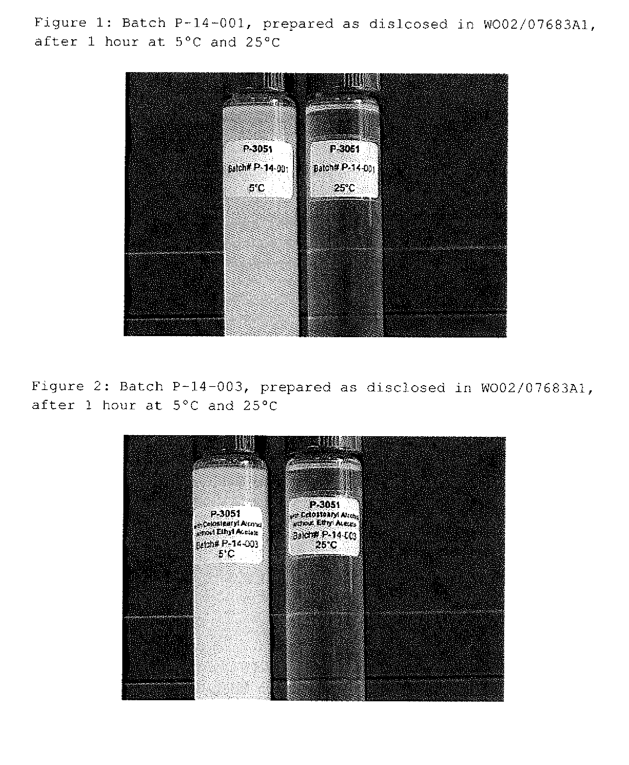

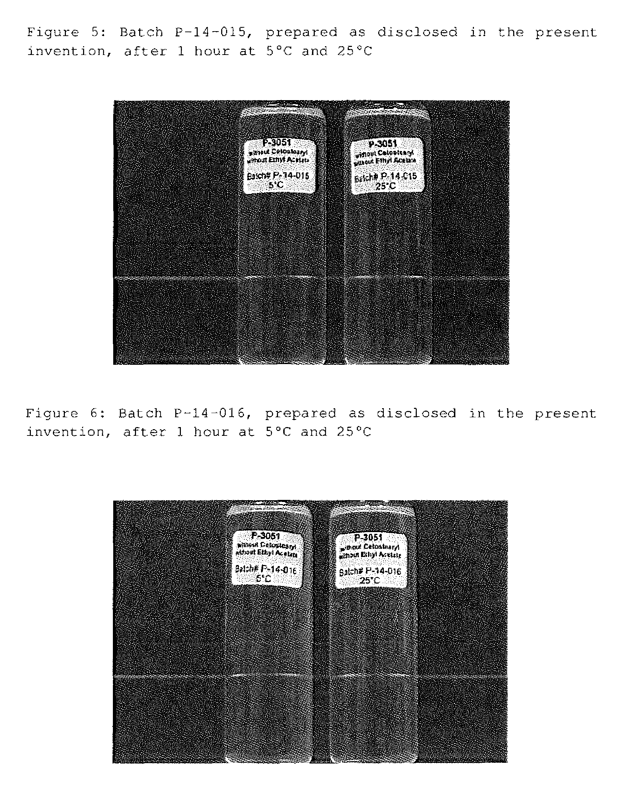

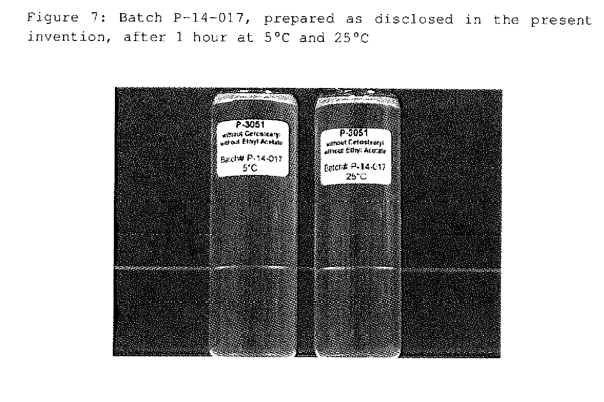

Pictures of the samples were taken before and after the exposure time at each temperature to evaluate the appearance of the solution and are reported in Figures 1 to 7. Observations are summarized in Table 1.

Table 1:

| Batch number | T=5°C | T=25°C |
|---|---|---|
| P-14-004 | Clear solution | Clear solution |
| P-14-014 | Clear solution | Clear solution |
| P-14-015 | Clear solution | Clear solution |
| P-14-016 | Clear solution | Clear solution |
| P-14-017 | Clear solution | Clear solution |
| P-14-003 | White flocculate | Clear solution |
| P-14-001 | White flocculate | Clear solution |

As it shall be easily appreciated, the solutions prepared according to the teaching of the present invention (batches P-14-004, P-14-014 and P-14-015) are superior to the solutions prepared following the disclosure of WO02/07683A1 (batch P-14-003) if exposed to temperatures below 10°C, since no white flocculate is observed. The absence of the white flocculate allows the formulations prepared following the teaching of the present invention to be transported without the need of a controlled temperature environment during the cold season.

EXAMPLE 4

The formulations prepared according to Example 1 (batches P-14-004, P-14-014 and P-14-015) and those prepared according to Example 2 (batches P-14-001, P-14-002 and P-14-003) were subjected to an accelerated stability study at a temperature higher than 40°C for one week in a controlled temperature storage chamber to evaluate the technological stability.

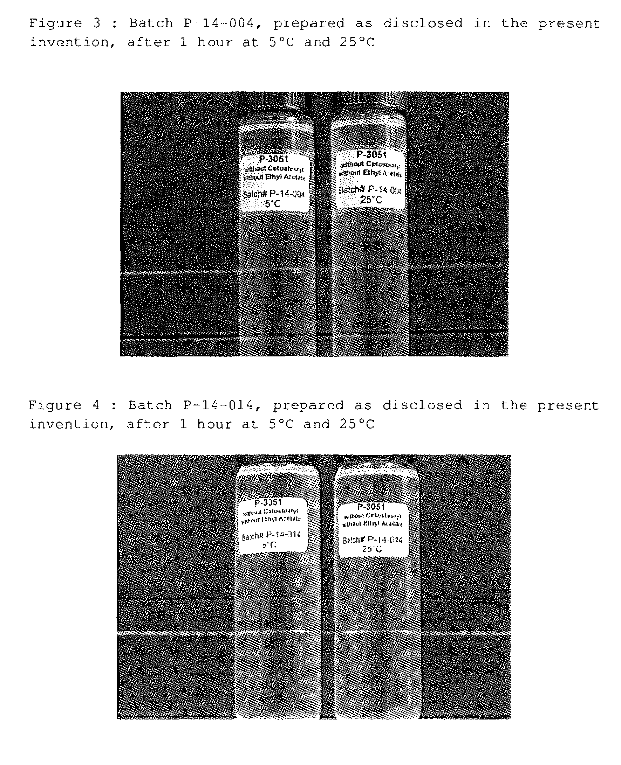

Pictures of the samples, which are reported in figures 3 and 4, were taken before and after the exposure time to evaluate the color of the solution, according to European Pharmacopoeia (monograph 2.2.2, method II, 7th Edition - 7.0) for the yellow series (Y) and the brown-yellow series (BY). According to the cited European Pharmacopoeia's monograph, colors of solutions are reported in 7-point scale, where Y1 corresponds to most intense yellow and Y2, Y3 etc. correspond to gradually less intense yellow, where Y7 is least yellow, and no yellow is comparable to water. Similarly, BY1 corresponds to most intense brown yellow and BY7 is less intense brown yellow. No brown yellow is comparable to water. Using identical tubes of colorless, transparent, neutral glass with a flat base and an internal diameter of 15 mm to 25 mm, the liquid to be examined was compared to water or the reference color solution. The colors were compared in diffused daylight, viewing vertically against a white background. The notation "<Y7 or <BY7", means that the color of solution is not appreciably different from the color of an equal amount of purified water.

Results are summarized in Table 2.

Table 2

| Batch number | t0 | t=2 weeks |
|---|---|---|
| P-14-004 | <Y7; <BY7 | <Y7; <BY7 |
| P-14-014 | <Y7; <BY7 | <Y7; <BY7 |
| P-14-015 | <Y7; <BY7 | <Y7; <BY7 |
| P-14-001 | <Y7; <BY7 | <Y7; <BY7 |
| P-14-002 | <Y7; <BY7 | <Y7; <BY7 |
| P-14-003 | <Y7; <BY7 | <Y7; <BY7 |

Conclusions. The solutions prepared following the teaching of the present invention (batches P-14-004, P-14-014 and P-14-015), although simpler, show a behaviour comparable to the solutions prepared following the disclosure of WO02/07683A1 (batch P-14-001, P-14-002 and batch P-14-003), if exposed to a temperature higher than 40°C, since no discoloration is observed. The absence of the discoloration at high temperature, together with the absence of flocculation at low temperatures, avoids the need, for the formulations prepared following the teaching of the present invention, to be stored at controlled temperature.

EXAMPLE 5 (Viscosity)

The formulations prepared according to the teaching of the present invention as per the Example 1 (batch P-14-004, P-14-014 and P-14-015) and the formulations prepared following the disclosure of WO02/07683A1 as per the Example 2 (batch P-14-001, P-14-002 and batch P-14-003) were subjected to an accelerated stability study at a temperature higher than 40°C for one week in a controlled temperature storage chamber to evaluate the technological stability.

Viscosity was determined using a suspended level viscometer size number 1, according to European Pharmacopoeia (7th edition, monograph 2.2.9), at a temperature of 25 $\pm$ 0.1 °C. The suspended level viscometer was filled in as described in the cited reference using an appropriate liquid quantity (approx. 17 mL).

The time required for the level of the liquid to drop from the mark E to the mark F was measured with a stopwatch; the average of three readings was used as the flow time of the liquid to be examined.

The kinematic viscosity η, expressed in millipascal x seconds (mPas) was calculated using the formula:

$$v = kt$$

where
k = constant of the viscometer, expressed in square millimetres per second squared and determined using a suitable viscometer calibration liquid
t = flow time, in seconds, of the liquid to be examined. Kinematic viscosity data collected at the starting point (t0, i.e. before exposure to a temperature higher than 40°C) were compared with the data obtained after 2 weeks of exposure at a temperature higher than 40°C in terms of percent difference.

For the purpose of this invention, an acceptable loss of viscosity means that the viscosity difference, calculated with reference to the starting point, should not exceed the value of 10%.

Results are summarized in Table 5.

Table 5

| Batch number | Kinematic viscosity (mPas) | % difference |
|---|---|---|
| | t0 | t=2 weeks |
| P-14-004 | 11.83 | - 0.83 |
| P-14-014 | 11.36 | -0.74 |
| P-14-015 | 5.65 | -0.59 |
| P-14-001 | 10. 65 | - 0.61 |
| P-14-002 | 10.88 | - 0.79 |
| P-14-003 | 12.04 | - 0.91 |

Conclusions. The formulations prepared following the teaching of the present invention (batch P-14-004, P-14-014 and P-14-015), although simpler, show a behaviour comparable to the formulations prepared following the disclosure of WO02/07683A1 (batch P-14-001, P-14-002 and batch P-14-003) if exposed to a temperature higher than 40°C, since an acceptable loss of viscosity is observed. The observed acceptable loss of viscosity leads to a superior technological stability.

EXAMPLE 6 (Drying Time)

The formulations prepared according to the teaching of the present invention as per the Example 1 (batches P-14-004, P-14-014 and P-14-015) and the formulations prepared following the disclosure of WO02/07683A1 as per the Example 2 (batch P-14-001, P-14-002 and batch P-14-003) were compared to evaluate the drying time once applied on the nails, i.e. the time taken by the solvent to evaporate to leave a dry surface. Evaporation time was calculated by measuring the weight loss over time of a glass slide following application of a given quantity of the formulation on a given surface, realized through a plastic hedge applied on the glass. Five microliters of formulation were applied on 2 cm$^2$ surface. Experiments were carried out at room temperature. Three measurements were taken for each batch and the mean value was used for the calculation. Evaporation time was reached when at least 80% of the start weight was lost. Results are summarized in Table 6.

Table 6: evaporation time

| Batch number | Evaporation time (seconds) |
|---|---|
| P-14-004 | 106 |
| P-14-014 | 90 |
| P-14-015 | 87 |
| P-14-003 | 123 |
| P-14-002 | 138 |
| P-14-001 | 129 |

From the results above the formulations prepared according to the teaching of the present invention (batch P-14-004, P-14-014 and P-14-015) are superior to the formulations prepared following the disclosure of WO02/07683A1 (batch P-14-001, P-14-002 and batch P-14-003) in that the drying time is shorter, thus realizing a user-friendly way of application: the user needs to wait a short time to let the formulation dry before using his/her hands/feet in usual daily operations.

Example 7 (preparation procedure)

The formulations prepared according to the teaching of the present invention as per the Example 1 (batch P-14-004, P-14-014, P-14-015, P-14-016 and P-14-017) and the formulations prepared following the disclosure of WO02/07683A1 as per the Example 2 (batch P-14-001, P-14-002 and batch P-14-003) were compared to evaluate the preparation process.
The formulations were prepared by mixing the ingredients using a suitable closed vessel provided with a stirrer.

The resulting mixture was stirred until a clear solution was obtained. The time (hours) required to obtain a clear solution was recorded. Results are summarized in Table 7

Table 7: time (hours) to obtain a clear solution.

| Batch number | Time to clear solution (hours) |
|---|---|
| P-14-004 | 2.5 |
| P-14-014 | 2.6 |
| P-14-015 | 2.3 |
| P-14-016 | 2.3 |
| P-14-017 | 2.3 |
| P-14-003 | 3.5 |
| P-14-002 | 3.5 |
| P-14-001 | 3.5 |

From the results above the formulations prepared according to the teaching of the present invention (batch P-14-004, P-14-014, P-14-015, P-14-016 and P-14-017) are superior to the formulations prepared following the disclosure of WO02/07683A1 (batch P-14-001, P-14-002 and batch P-14-003). The dissolution step of the waxy solid cetostearyl alcohol, included in the formulations prepared following the disclosure of WO02/07683A1, increased the preparation time to a clear solution of about 1 hour. Thus, the formulations prepared according to the teaching of the present invention permit to realize a more economical, less time consuming, environment friendly production process.

**Claims**

1. A composition comprising at least about 7% by weight ciclopirox or a pharmaceutically acceptable salt thereof, hydroxypropyl chitosan, a lower alkanol and water.

2. The composition according to claim 1, wherein ciclopirox or a pharmaceutically acceptable salt thereof is present in an amount from 7% to 9% by weight.

3. The composition according to claim 1, wherein said lower alkanol is ethanol.

4. The composition according to claim 1, wherein said pharmaceutically acceptable salt is ciclopirox olamine.

5. A composition according to claim 1, consisting essentially of:

    a) ciclopirox and/or at least a pharmaceutically acceptable salt thereof in an amount of from 7 to 9% by weight of the composition,
    b) hydroxypropyl chitosan in an amount of from 0.1 to 4% by weight of the composition,
    c) water in an amount of from 10.0 to 30.0% by weight of the composition,
    d) at least a lower alkanol in an amount of from 65 to 85% by weight of the composition.

6. The composition of claim 5, wherein component b) is present in an amount from 0.2 to 2.0% by weight of the composition.

7. The composition of claim 5, wherein component c) is present in an amount from 12 to 20% by weight of the composition.

8. The composition of claim 5, wherein component d) is selected from ethanol, propanol, isopropanol, butanol and mixtures thereof.

9. The composition of claim 8, wherein component d) is ethanol.

10. The composition of claim 5 consisting of a) 7.5 to 8.5% by weight ciclopirox, b) 0.2 to 2.0% by weight hydroxypropyl chitosan, c) 12 to 20% by weight purified water and d) 70.0 to 80.0% by weight ethanol

11. The composition according to any of the preceding claims in the form of a nail lacquer.

12. The composition according to any of the preceding claims, for use in the treatment of onychomycosis.

13. The composition for use according to claim 12, **characterized in that** it is applied on infected nails.

14. The composition for use according to claim 13, **characterized in that** it is applied once-daily.

15. The composition for use according to claim 13, **characterized in that** it is applied less than once-daily.

Figure 1: Batch P-14-001, prepared as dislcosed in WO02/07683A1,
after 1 hour at 5°C and 25°C

Figure 2: Batch P-14-003, prepared as disclosed in WO02/07683A1,
after 1 hour at 5°C and 25°C

Figure 3 : Batch P-14-004, prepared as disclosed in the present invention, after 1 hour at 5°C and 25°C

Figure 4 : Batch P-14-014, prepared as disclosed in the present invention, after 1 hour at 5°C and 25°C

Figure 5: Batch P-14-015, prepared as disclosed in the present invention, after 1 hour at 5°C and 25°C

Figure 6: Batch P-14-016, prepared as disclosed in the present invention, after 1 hour at 5°C and 25°C

Figure 7: Batch P-14-017, prepared as disclosed in the present invention, after 1 hour at 5°C and 25°C

Europäisches
Patentamt

European
Patent Office

Office européen
des brevets

# EUROPEAN SEARCH REPORT

Application Number

EP 14 15 6113

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 02/07683 A1 (POLICHEM SA [LU]; MAILLAND FEDERICO [IT]) 31 January 2002 (2002-01-31) | 1-15 | INV. A61K31/4418 A61K47/10 |
| Y | * claims 1,4,6-9 * <br> * page 6, paragraph 1-3 * <br> * page 10, paragraph 1 * <br> * examples 1, 2 * | 1-15 | A61K47/36 A61K9/08 A61P31/10 |
| X | WO 2008/098869 A2 (POLICHEM SA [LU]; MR FEDERICO MAILLAND [IT]) 21 August 2008 (2008-08-21) | 1-6,8,9, 12,13 | |
| Y | * examples 2, 3 * | 1-15 | |
| Y,D | MONTI D ET AL: "In vitro transungual permeation of ciclopirox from a hydroxypropyl chitosan-based, water-soluble nail lacquer", DRUG DEVELOPMENT AND INDUSTRIAL PHARMACY, NEW YORK, NY, US, vol. 31, no. 1, January 2005 (2005-01), pages 11-17, XP008082561, ISSN: 0363-9045 * abstract * * page 15, column 2, paragraph 2 * | 1-15 | |
| Y,D | BARAN R ET AL: "An innovative water-soluble biopolymer improves efficacy of ciclopirox nail lacquer in the management of onychomycosis", JOURNAL OF THE EUROPEAN ACADEMY OF DERMATOLOGY AND VENEREOLOGY, vol. 23, no. 7, July 2009 (2009-07), pages 773-781, XP055113869, ISSN: 0926-9959, DOI: 10.1111/j.1468-3083.2009.03164.x * abstract * * figures 1, 3 * | 1-15 | |

TECHNICAL FIELDS SEARCHED (IPC)

A61K

-/--

The present search report has been drawn up for all claims

| Place of search | Date of completion of the search | Examiner |
|---|---|---|
| Munich | 15 April 2014 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 14 15 6113

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | SUBISSI ALESSANDRO ET AL: "Ciclopirox: recent nonclinical and clinical data relevant to its use as a topical antimycotic agent.", DRUGS 12 NOV 2010, vol. 70, no. 16, 12 November 2010 (2010-11-12), pages 2133-2152, XP009177577, ISSN: 0012-6667 * abstract * * page 2040, column 2, paragraph 3 - page 2041, column 1, paragraph 1 * * table VI * | 1-15 | |
| Y | GUPTA A K ET AL: "Therapies for the treatment of onychomycosis", CLINICS IN DERMATOLOGY, vol. 31, no. 5, September 2013 (2013-09), pages 544-554, XP055113871, ISSN: 0738-081X, DOI: 10.1016/j.clindermatol.2013.06.011 * page 546, column 2, paragraph 6 - page 547, column 1, paragraph 5 * | 1-15 | |
| A | BOHN M ET AL: "Dermatopharmacology of ciclopirox nail lacquer topical solution 8% in the treatment of onychomycosis", JOURNAL OF THE AMERICAN ACADEMY OF DERMATOLOGY, vol. 43, no. 4 Suppl, October 2000 (2000-10), pages S57-S69, XP055113868, ISSN: 0190-9622, DOI: 10.1067/mjd.2000.109072 * the whole document * | 1-15 | |

TECHNICAL FIELDS SEARCHED (IPC)

The present search report has been drawn up for all claims

| Place of search | Date of completion of the search | Examiner |
|---|---|---|
| Munich | 15 April 2014 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 14 15 6113

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2014

| Patent document cited in search report | | Publication date | Patent family member(s) | | | Publication date |
|---|---|---|---|---|---|---|
| WO 0207683 | A1 | 31-01-2002 | AT | 261296 | T | 15-03-2004 |
| | | | AU | 7255101 | A | 05-02-2002 |
| | | | BG | 65953 | B1 | 30-07-2010 |
| | | | BR | 0112725 | A | 02-09-2003 |
| | | | CA | 2416823 | A1 | 31-01-2002 |
| | | | CN | 1449273 | A | 15-10-2003 |
| | | | CN | 1679472 | A | 12-10-2005 |
| | | | CZ | 20030217 | A3 | 18-06-2003 |
| | | | DE | 10035991 | A1 | 14-02-2002 |
| | | | DE | 60102302 | D1 | 15-04-2004 |
| | | | DE | 60102302 | T2 | 29-07-2004 |
| | | | DK | 1303249 | T3 | 14-06-2004 |
| | | | EP | 1303249 | A1 | 23-04-2003 |
| | | | ES | 2215918 | T3 | 16-10-2004 |
| | | | HK | 1053067 | A1 | 06-08-2004 |
| | | | HR | P20030037 | A2 | 29-02-2004 |
| | | | HU | 0301496 | A2 | 28-08-2003 |
| | | | IL | 154078 | A | 17-02-2010 |
| | | | IL | 193224 | A | 31-12-2013 |
| | | | JP | 4170751 | B2 | 22-10-2008 |
| | | | JP | 2004504333 | A | 12-02-2004 |
| | | | JP | 2007153910 | A | 21-06-2007 |
| | | | JP | 2008044957 | A | 28-02-2008 |
| | | | KR | 20030036637 | A | 09-05-2003 |
| | | | MX | PA03000764 | A | 04-06-2003 |
| | | | PL | 363070 | A1 | 15-11-2004 |
| | | | PT | 1303249 | E | 30-06-2004 |
| | | | SI | 1303249 | T1 | 31-08-2004 |
| | | | SK | 1022003 | A3 | 07-10-2003 |
| | | | TR | 200400612 | T4 | 21-04-2004 |
| | | | US | 2004022831 | A1 | 05-02-2004 |
| | | | WO | 0207683 | A1 | 31-01-2002 |
| | | | YU | 4503 | A | 03-03-2006 |
| WO 2008098869 | A2 | 21-08-2008 | AR | 065316 | A1 | 27-05-2009 |
| | | | AT | 522221 | T | 15-09-2011 |
| | | | AU | 2008214693 | A1 | 21-08-2008 |
| | | | CA | 2677715 | A1 | 21-08-2008 |
| | | | CL | 4442008 | A1 | 22-08-2008 |
| | | | CN | 101663039 | A | 03-03-2010 |
| | | | CN | 102988405 | A | 27-03-2013 |
| | | | CO | 6230979 | A2 | 20-12-2010 |
| | | | DK | 2117564 | T3 | 17-10-2011 |
| | | | DK | 2377541 | T3 | 11-11-2013 |
| | | | EA | 200901093 | A1 | 26-02-2010 |
| | | | EA | 201270116 | A1 | 29-06-2012 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 14 15 6113

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2014

| Patent document cited in search report | Publication date | Patent family member(s) | | Publication date |
|---|---|---|---|---|
| | | EP | 1958638 A1 | 20-08-2008 |
| | | EP | 2117564 A2 | 18-11-2009 |
| | | EP | 2377541 A1 | 19-10-2011 |
| | | ES | 2369116 T3 | 25-11-2011 |
| | | ES | 2436647 T3 | 03-01-2014 |
| | | HK | 1133189 A1 | 23-12-2011 |
| | | HR | P20110667 T1 | 31-10-2011 |
| | | HR | P20131175 T1 | 17-01-2014 |
| | | JP | 5426401 B2 | 26-02-2014 |
| | | JP | 2010518138 A | 27-05-2010 |
| | | JP | 2013213048 A | 17-10-2013 |
| | | KR | 20090121312 A | 25-11-2009 |
| | | KR | 20110075043 A | 05-07-2011 |
| | | MA | 31236 B1 | 01-03-2010 |
| | | NZ | 578896 A | 24-02-2012 |
| | | PT | 2117564 E | 17-10-2011 |
| | | PT | 2377541 E | 21-11-2013 |
| | | RS | 52031 B | 30-04-2012 |
| | | SI | 2117564 T1 | 30-12-2011 |
| | | SI | 2377541 T1 | 31-01-2014 |
| | | US | 2010152133 A1 | 17-06-2010 |
| | | WO | 2008098869 A2 | 21-08-2008 |
| | | ZA | 200905443 A | 27-10-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0015202 A
- WO 9939680 A
- EP 226984 A

- WO 020768 A1
- WO 0207683 A1

### Non-patent literature cited in the description

- Epidemiology. Onychomycosis - the current approach to diagnosis and therapy. Martin Dunitz, 1999, 6-9
- **TOSTI A ; HAY R ; ARENAS-GUZMÁN R.** Patients at risk of onychomycosis - risk factor identification and active prevention. *J Eur Acad Dermatol Veneorol,* 2005, vol. 19, 13-16
- **AJIT C ; SUVANNASANKHA A ; ZAERI N ; MUNOZ SJ.** Terbinafine-associated hepatotoxicity. *Am J Med Sci.,* 2003, vol. 325, 292-5
- **SLØRDAL L ; SPIGSET O.** Heart failure induced by non-cardiac drugs. *Drug Saf.,* 2006, vol. 29, 567-86
- **ELEWSKI B ; GHANNOUM MA ; MAYSER P et al.** Efficacy, safety and tolerability of topical terbinafine nail solution in patients with mild-to-moderate toenail onychomycosis: results from three randomized studies using double-blind vehicle-controlled and open-label active-controlled designs. *J Eur Acad Dermatol Veneorol,* 2011

- **SUBISSI A. et al.** Ciclopirox - Recent Nonclinical and Clinical Data Relevant to its Use as a Topical Antimycotic Agent. *Drugs,* 2010, vol. 70 (16), 2133-2152
- **GUPTA AK ; MALKIN KF.** Ciclopirox nail lacquer and podiatric practice. *J Am Podiatr Med Assoc,* 2000, vol. 90 (10), 502-7
- **MONTI D ; SACCOMANI L ; CHETONI P et al.** *Drug Dev Ind Pharm.,* January 2005, vol. 31 (1), 11-7
- **BARAN R ; TOSTI A ; HARTMANE I et al.** An innovative water soluble biopolymer improves efficacy of ciclopirox nail lacquer in the management of onychomycosis. *J Eur Acad Dermatol Veneorol,* 2009, vol. 23, 773-781